Europäisches Patentamt

European Patent Office

Office européen des brevets

(11) Publication number: **0 569 833 A2**

## EUROPEAN PATENT APPLICATION

(21) Application number: **93107259.9**

(22) Date of filing: **05.05.93**

(51) Int. Cl.5: **C12Q 1/68**, C07H 21/04, C12P 19/34

(30) Priority: **15.05.92 US 883660**

(43) Date of publication of application:
**18.11.93 Bulletin 93/46**

(84) Designated Contracting States:
**BE CH DE DK ES FR GB GR IE IT LI LU NL PT**

(71) Applicant: **F. HOFFMANN-LA ROCHE & CO.
Aktiengesellschaft
Postfach 3255
CH-4002 Basel(CH)**

(72) Inventor: **Reynolds, Rebecca
913 Independence Drive
Alameda, California 94501(US)**

(74) Representative: **Wächter, Dieter Ernst, Dr. et
al
P.O. Box 3255
CH-4002 Basel (CH)**

(54) **Gender test Method.**

(57) The present invention relates to a method for determining the gender of a human being based on a DNA sample originating from said human being. In one embodiment of the invention, the oligonucleotide primers CTGGAGAGCCACAAGCTGAC (SEQ ID NO:1); and TTGCTGTGGACTGCCAAGAG (SEQ ID NO:2) are used to amplify an approximately 209 base pair conserved region of the X and Y zinc finger protein coding sequence. Then, digestion of the amplified product with a HaeIII restriction enzyme results in distinguishable fragments for female and male samples. The invention also relates to the said oligonucleotide primers per se, preferably in labeled form, to the use of said primers as a diagnostic tool and to a test-kit comprising said oligonucleotide primers.

EP 0 569 833 A2

The present invention relates to a gender identification method and to specific probes and primers used in said method.

Gender determination is an important aspect of many scientific processes including forensic analysis of biological samples and the sexing of preimplantation embryos to determine the probability for sex-linked disorders with in vitro fertilization. Present gender determination analyses depend on the heteromorphic (XY) nature of the male. Thus, e.g. a sex determining gene, known as the testes-determining factor (TDF) is believed to be located in the genome of the heteromorphic individuals, and is believed to cause the bipotential gonadal primordia to differentiate into testes [Page, D.C. et al., Cell 51, 1091-1104 (1987)]. Therefore, TDF is the basis for many known gender determination tests.

A further gender determination method is based on the "dosage/X-inactivation" model described by Ferguson-Smith, M.A. in Brit. Med. J. 297, 635-636 (1988). More precisely, this method is based on the premise that the ZFY gene and a related gene on the X chromosome produce functionally interchangeable proteins. Thus, XY cells would have two active copies of the gene. On the other hand XX cells would have only one active copy of the gene due to "X-inactivation". Therefore, embryos which have two active copies of the gene would develop as males, while those with one active copy of the gene would develop as females.

The "dosage/X-inactivation" model was contradicted by Schneider-Gadicke, A. et al. [Cell 57, 1247-1258 (1989)]. Schneider-Gädicke cloned the human X homolog of the ZFY gene, designated ZFX, and found that this X chromosomal gene encodes a protein with a zinc finger domain closely related to that of the ZFY protein. By transcription analysis of human-rodent hybrid cell lines, it was found that ZFX escapes "X-inactivation".

Another gender determination method utilizes universal primers (i.e. primers which are useful for multiple species, such as e.g. humans, cattle, sheep and goats) and PCR to amplify 447 base pair (bp) or 445 bp fragments from male or female genomic DNA corresponding to the ZFY and ZFX genes [Aasen, E. and Medrano, J.F., Bio/Technology 8, 1279-1281 (1990)]. Then, restriction fragment length polymorphism (RFLP) analysis of the fragments, showed specific banding patterns between the sexes in the four species based on digestion with a panel of restriction enzymes (AluI, EcoRI, HaeIII, HinfI, MspI, PstI, SacI and SalI). In humans, the RFLP analysis revealed that the ZFX fragment had a single HaeIII site producing 400 bp and 45 bp fragments in both males and females, whereas the ZFY fragment had two HaeIII sites, thus yielding 317 bp, 84 bp and 46 bp fragments. Therefore, human male samples are identified by four fragments of 400, 317, 84 and 46/45 base pairs.

Also, a PCR-based diagnosis of sex chromosome aneuploidy has been described by Mutter, G.L. and Pomponio, R.J. in Nucleic Acids Research 19, 4203-4207 (1991). The method uses a single primer set directed against homologous but distinct genes on the X and Y chromosomes, and produces a 406 base pair product with sex chromosome specific restriction fragment length polymorphisms. The method shows sex chromosome imbalances when both X and Y chromosomes are present.

By using the known methods for gender identification there is alway a chance for ambriguous or incorrect results. The problem to be solved by the present invention was to provide a more accurate gender identification method than those presently in use.

The method for gender determination of the present invention utilizes the polymerase chain reaction (PCR) with specific primers to a region of the human X and Y chromosomes. Then, specific polymorphic restriction sites are utilized to distinguish male samples from female samples. In one embodiment, the present invention relates to a method for determining the gender of a human being based on a DNA sample originating from said human being, which method comprises the steps of: (a) amplifying a product from the sample using PCR with primers RR10 (CTGGAGAGCCACAAGCTGAC) (SEQ ID NO:1) and RR12 (TTGCTGTGGACTGCCAAGAG) (SEQ ID NO:2); (b) digesting the product with a HaeIII restriction enzyme; and (c) visualizing the digested product. A product of about 209 bp results from the amplification step. The HaeIII digestion then yields fragments of about 172 bp and about 37 bp if the sample was from a female, and fragments of about 172 bp, about 37 bp, about 88 bp and about 84 bp if the sample is from a male. The 88 bp and 84 bp fragments, if visualized on a gel, appear as either a single band or as a doublet band.

In another embodiment, the gender determination method comprises the steps of: (a) amplifying a 209 bp product which has the nucleotide sequence

```
CTGGAGAGCC ACAAGCTGAC CAGCAAGGCA GAGAAGGCCA TTGAATGTGA
TGAGTGTGGG AAGCATTTTT CTCATGCAGG GGCTTTGTTT ACTCACAAAA
TGGTGCATAA GGAAAAGGG GCCAACAAAA TGCACAAGTG TAAATTCTGT
GAATATGAGA CAGCTGAACA GGGGTTATTG AATCGCCACC TCTTGGCAGT
CCACAGCAA
```

[SEQ ID No: 3]

in a human DNA sample; (b) digesting the product with a HaeIII restriction enzyme; and (c) visualizing the digested product. This method envisions the use of other amplification procedures in addition to PCR. Examples of such other amplification procedures include ligase chain reaction (LCR) and polymerase ligase chain reaction (PLCR).

In a further embodiment, the invention is used in a reverse dot blot format for gender determination. In this format, immobilized probes to different areas of the amplified, labelled product indicate the presence or absence of male and female DNA in the sample, or the relative proportions of male and female DNA in a mixed sample.

The various objects, advantages and novel features of the invention will be more readily appreciated from the following detailed description when read in conjunction with the appended figures in which:

Fig. 1 is a photograph of an acrylamide gel of the HaeIII digestion of ZFX/ZFY products.

Fig. 2 is a representation of the reverse dot blot profiles of various mixed and unmixed male and female DNA samples (for further details see below).

Fig. 3A and Fig. 3B are photographs of acrylamide gels of the HaeIII digestion of mixed samples of female and male DNA (for further details see below).

The present invention relates to a method for determining the gender of a human being based on a biological sample comprising DNA from said human being. To facilitate understanding of the invention, a number of terms are defined below.

The term "gene" refers to a DNA sequence that comprises control and coding sequences necessary for the production of a recoverable bioactive polypeptide or precursor. The polypeptide can be encoded by a full length coding sequence or by any portion of the coding sequence, as long as its bioactivity (e.g. its enzymatic activity) is retained.

The term "oligonucleotide" as used herein is defined as a molecule comprised of two or more deoxyribonucleotides or ribonucleotides, preferably more than three, and usually more than ten. The exact size will depend on many factors, which in turn depends on the ultimate function or use of the oligonucleotide. The oligonucleotide may be derived synthetically or by cloning.

The term "primer" as used herein refers to an oligonucleotide which is capable of acting as a point of initiation of synthesis when placed under conditions in which primer extension is initiated. An oligonucleotide "primer" may occur naturally, as in a purified restriction digest or be produced synthetically. Synthesis of a primer extension product which is complementary to a nucleic acid strand is initiated in the presence of reagents needed for the amplification reaction, viz. four different nucleoside triphosphates and a thermostable polymerase enzyme in an appropriate buffer at a suitable temperature. A "buffer" includes cofactors (such as divalent metal ions) and salt (to provide the appropriate ionic strength), adjusted to the desired pH.

A primer is single-stranded for maximum efficiency in amplification, but may alternatively be double-stranded. If double-stranded, the primer is first treated to separate its strands before being used to prepare extension products. The primer is usually an oligodeoxyribonucleotide. The primer must be sufficiently long to prime the synthesis of extension products in the presence of the polymerase enzyme. The exact length of a primer will depend on many factors, such as source of primer and result desired, and the reaction temperature must be adjusted depending on primer length and nucleotide sequence to ensure proper annealing of primer to template. Depending on the complexity of the target sequence, an oligonucleotide primer typically contains 15 to 35 nucleotides. Short primer molecules generally require lower temperatures to form sufficiently stable complexes with template.

A primer is selected to be "substantially" complementary to a strand of specific sequence of the template. A primer must be sufficiently complementary to hybridize with a template strand for primer elongation to occur. A primer sequence need not reflect the exact sequence of the template. For example, a non-complementary nucleotide fragment may be attached to the 5' end of the primer, with the remainder of

3

the primer sequence being substantially complementary to the strand. Non-complementary bases or longer sequences can be interspersed into the primer, provided that the primer sequence has sufficient complementarity with the sequence of the template to hybridize and thereby form a template primer complex for synthesis of the extension product of the primer.

The terms "restriction endonucleases" and "restriction enzymes" refer to bacterial enzymes which cut double-stranded DNA at or near a specific nucleotide sequence.

Furthermore, the present invention relates to the above-mentioned primers RR10 and RR12 per se, preferably in labeled form, to the use of said primers as a diagnostic tool and to a test-Kit comprising said oligonucleotide primers and preferably the reagents needed for the amplification reaction.

As noted above, gender determination of a human biological sample is very important in forensic investigations. The present invention is such a gender determination method which provides numerous advantages over known methods.

The advantages of the present invention derive from the identification of a conserved region of the human X and Y zinc finger proteins with HaeIII sites common to the X and Y chromosomes and a polymorphic HaeIII site that is present only in the Y chromosome. This conserved region is a relatively small region (about 209 bp). As explained in greater detail below, the presence of the HaeIII restriction sites is an important factor in the method, and thus the conserved region could be reduced or enlarged so long as the HaeIII sites are present and the fragments that result from the HaeIII digestion are discernible.

When the 209 bp conserved region is digested by HaeIII restriction enzymes, it will yield discernible fragments of 37 bp (nucleotides 1-37) and 172 bp (nucleotides 38-209) from the X chromosome. If the conserved region is from a female (XX) then only these two fragments will be observed. However, if the conserved region is from a male (XY), HaeIII digestion yields discernible fragments of 172 bp, 37 bp, 88 bp and 84 bp.

The X chromosome has only a single HaeIII restriction site on the 209 bp conserved region, thus splitting the conserved region into fragments of 172 bp and 37 bp. However, the Y chromosome has an additional HaeIII restriction site in the 172 bp fragment, thus further splitting that fragment into an 88 bp fragment and 84 bp fragment. Therefore, the basis for the differentiation of male and female biological samples in the present invention is the additional HaeIII site in the conserved region of a Y chromosome.

The most common use envisioned for the present invention is in forensic analysis where the samples to be analyzed are often very small. Therefore, an amplification step is often necessary to create enough product (conserved region) to yield discernible fragments upon HaeIII digestion.

Suitable amplification techniques for the human biological sample include PCR, LCR and PLCR, with PCR being the preferred technique. PCR is a well-known amplification technique which is described in United States Patent Nos. 4,683,195 and 4,683,202, both of which are incorporated herein by reference. Briefly, the PCR amplification process comprises the steps of:

(a) contacting each nucleic acid strand in the sample with four different nucleoside triphosphates and two oligonucleotide primers for each specific sequence being amplified (in this case the 209 bp conserved region), wherein each primer is selected to be substantially complementary to the different stands of the specific sequence, such that the extension product synthesized from one primer, when separated from its complement, can serve as a template for synthesis of the extension product of the other primer, said contacting being at a temperature that allows hybridization of each primer to a complementary nucleic acid strand;

(b) contacting each nucleic acid strand, at the same time as or after step (a), with a thermostable DNA polymerase that enables combination of the nucleoside triphosphates to form primer extension products complementary to each strand of the specific nucleic acid sequence;

(c) maintaining the mixture from step (b) at an effective temperature for an effective time to promote the activity of the enzyme and to synthesize, for each different sequence being amplified, an extension product of each primer that is complementary to each nucleic acid strand template, but not so high as to separate each extension product from the complementary strand template;

(d) heating the mixture from step (c) for an effective time and at an effective temperature to separate the primer extension products from the templates on which they were synthesized to produce single-stranded molecules but not so high as to denature irreversibly the enzyme;

(e) cooling the mixture from step (d) for an effective time and to an effective temperature to promote hybridization of a primer to each of the single-stranded molecules produced in step (d); and

(f) maintaining the mixture from step (e) at an effective temperature for an effective time to promote the activity of the enzyme and to synthesize, for each different sequence being amplified, an extension product of each primer that is complementary to each nucleic acid template produced in step (d) but not so high as to separate each extension product from the complementary strand template. The effective times and temperatures in steps (e) and (f) may coincide, so that steps (e) and (f) can be carried out simultaneously. Steps (d)-(f) are repeated until the desired level of amplification is obtained.

In the present invention, the two primers that are used in the PCR process are:

(1) RR10

CTGGAGAGCCACAAGCTGAC (SEQ ID NO: 1)

and

(2) RR12

TTGCTGTGGACTGCCAAGAG (SEQ ID NO: 2).

Oligonucleotide primers such as RR10 and RR12 can be prepared using any suitable method, such as, for example, the phosphotriester and phosphodiester methods or automated embodiments thereof. In one such automated embodiment, diethylphosphoramidites are used as starting materials and can be synthesized as described by Beaucage et al., 1981, Tetrahedron Letters 22:1859-1862. One method for synthesizing oligonucleotides on a modified solid support is described in U.S. Patent No. 4,458,066 which is also incorporated herein by reference. One can also use a primer that has been isolated from a biological source (such as a restriction endonuclease digest).

Applying PCR amplification to a human biological sample using the RR10 primer (SEQ ID NO: 1) and the RR12 primer (SEQ ID NO: 2) yields a 209 bp product (SEQ ID NO:3), provided there is sufficient sample for the PCR process. Presently, it is preferable to have at least 0.5 ng of DNA sample to obtain unambiguous confirmation of the male gender, and from 1 to 2 ng of DNA sample to obtain unambiguous confirmation of the female gender. This small amount of DNA sample is favorably contrasted with the requirement of over 250 ng of DNA sample necessary for some well-known gender identification techniques, such as the sex identification techniques taught by Akane, A. et al. in Forensic Science International 49, 81 (1991). Also, the small 209 bp product has been found to be unaffected by even severely degraded forensic samples, whereas a larger product would be more susceptible to damage in a degraded sample.

As stated above, other amplification techniques such as LCR and PLCR may also be used in the present invention. Briefly, ligase chain reaction (LCR) utilizes adjacent primers and a thermostable ligase to join those primers after they have hybridized to a complementary strand of DNA. (see Landegren, U. et al., Science 241, 1077 (1988) and Barany, F., Proc. Natl. Acad. Sci. U.S.A. 88, 189 (1991) both of which are incorporated herein by reference.) Oligonucleotide products are exponentially amplified by thermal cycling of the ligation reaction in the presence of a second set of adjacent oligonucleotides, complementary to the first set and the target. Preferably, the oligonucleotides should be about 20 to 25 nucleotides long for proper hybridization. Also, specificity of the ligation is enhanced by performing the reaction at or near the melting temperature of the two oligonucleotides. The exponential amplification of product results as the ligation products of one cycle become the targets for the next cycle of ligation. A suitable thermostable ligase can be isolated from the plasmid libraries of Thermus aquaticus strain HB8 DNA (available e.g. from the American Type Culture Collection under the accession number ATCC 27634) as taught by Barany, F. in Proc. Natl. Acad. Sci. U.S.A. 88, 189 (1991). Oligonucleotides for ligation are prepared in the same manner the primers used in the PCR described above. Each cycle of LCR comprises incubation of the oligonucleotides in the presence of target DNA sample and T. aquaticus ligase at about 94°C for about 1 minute followed by 65°C for about 4 minutes. Generally, 20 to 30 cycles produces sufficient amounts of DNA for gender determination using the present invention.

As its name suggests, polymerase ligase chain reaction (PLCR) combines features of PCR with features of ligase chain reaction (LCR). PLCR was developed in part as a technique to increase the specificity of allele-specific PCR in which the low concentrations of dNTP utilized (~1 $\mu$M) limited the extent of amplification. In PLCR, DNA is denatured and four complementary, but not adjacent, oligonucleotide primers are added with dNTPs, a thermostable DNA polymerase and a thermostable ligase.

The primers anneal to target DNA in a non-adjacent fashion and the thermostable DNA polymerase causes the addition of appropriate dNTPs to the 3' end of the downstream primer to fill the gap between the non-adjacent primers and thus render the primers adjacent. The thermostable ligase will then ligate the two adjacent oligonucleotide primers.

Following the amplification step using any appropriate technique, the amplified product (conserved region) is isolated and subjected to HaeIII digestion. The HaeIII restriction endonuclease may be obtained from New England Biolabs as well as other sources. Generally, about 0.5 $\mu$l of HaeIII enzyme at 10 U/$\mu$l is used to digest between about 5 $\mu$l and about 10 $\mu$l of PCR product. The digestion generally takes place at about 37°C for between 2 hours and overnight. HaeIII buffer (also obtainable from New England Biolabs [NEB], Beverly MA, U.S.A.) is also often utilized in the digestion step.

Following digestion of the product, the fragments must be visualized in order to determine the digestion profile. Many visualization techniques are available, including gel electrophoresis, isoelectric focussing, high performance liquid chromotography (HPLC), and gene scanner if the primers are labelled with fluorescent tags.

The preferred method of visualizing the HaeIII digestion fragments is by gel electrophoresis using either an agarose gel (preferably a 3 % NuSieve/1 % agarose gel) or a 9 % acrylamide gel. Use of the 9 % acrylamide gel is the most preferred method. The HaeIII digested product is mixed with about 2 $\mu$l of sample dye loading buffer, and then, the mixture is loaded onto the gel. The gel is run in 1 x TBE (0.089 M Tris/borate, 2 mM EDTA), and at least one undigested sample should also be run to indicate complete product digestion.

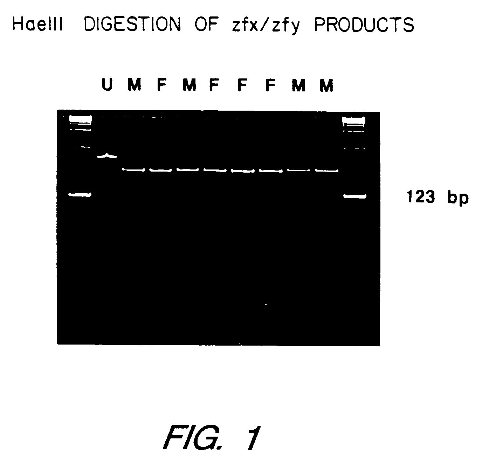

As shown in Fig. 1, visualization of the RR10/RR12 amplified product on a gel yields bands at 172 bp and at 37 bp. However, samples of male origin will have additional bands at 88 bp and 84 bp. These Y-specific digestion products appear as a single band on the 3 % NuSieve/1 % agarose gel and as a doublet on the 9 % acrylamide gel. Fig. 1 shows an acrylamide gel, and thus the male samples show the 88/84 bp doublet.

The present invention is a very sensitive assay for mixed samples from two or more individuals. Specifically, it has been found that use of the present invention permits detection of male DNA in a mixture in which the male DNA is present at 1/10 the amount of the female DNA.

However, as might be expected, female DNA contaminating a male DNA sample to the same degree (i.e. 1/10) cannot be distinguished, since males also contain an X chromosome. Specifically, once there is a 5-fold or greater excess of male DNA over female DNA, the mixed sample looks essentially like an unmixed male DNA sample.

An equal mixture of female and male DNA can be distinguished visually from a pure male DNA sample by noting the relative intensities of the 88/84 bp doublet and the 172 bp fragment from which it is derived. In an unmixed male DNA sample, there are an equal number of X and Y chromosomes, so the intensities of the 172 bp fragment and 88/84 bp doublet should be comparable. In a mixture containing equal amounts of female and male DNA, there are 3 X chromosomes for every 1 Y chromosome, and therefore, the 172 bp band will be noticeably darker than the doublet. These differences may be quantitated by using fluorescently labelled primers and an appropriate scanner, such as e.g. a GeneScanner or by simply scanning a negative of a photograph of the gel.

Another technique for visualization of the amplified product to determine gender is a reverse dot blot analysis. This is a well-known technique in which a label or indicator is attached to the amplified product, which is then exposed to an immobilized probe. If the probe and product are complementary, then the probe and product hybridize, and the presence of the desired product is shown by activation of the indicator or label on the product. An embodiment of the reverse dot blot technique is explained in greater detail by Saiki, R. et al. in Proc. Natl. Acad. Sci. USA 86, 6230-6234 (1989), incorporated herein by reference.

Generally, the probe is immobilized on a solid support such as a nylon membrane by means of a poly (dT) tail added using terminal transferase or added during chemical synthesis of the probe. Also, the amplified product is generally biotinylated, and thus will bind an avidin horseradish peroxidase conjugate. The horseradish peroxidase will then convert a colorless dye into a colored precipitate to indicate the presence of the desired product in the sample.

In a preferred embodiment of the present invention, biotinylated primers RR10 and RR12 are used to produce a 209 bp biotinylated amplified product by the PCR process. Then, the product is exposed to three immobilized probes: (1) a C probe (common to both X and Y chromosomes) comprising a nucleotide sequence AGAGAAGGCC ATTGAATGT (SEQ ID NO:4) complementary to a region of the product represented by nucleotides 30-48 of SEQ ID NO:3; (2) an X probe comprising a nucleotide sequence GAAAAAGGAG CCAACAAAAT (SEQ ID NO:5) complementary to a region of the product represented by nucleotides 112-131 of SEQ ID NO:3 wherein nucleotide 120 is an A residue, thus indicating the lack of a HaeIII site; and (3) a Y probe comprising a nucleotide sequence ATTTTGTTGG CCCCTTTTTC (SEQ ID NO:6) complementary to a region of the product represented by nucleotides 112-131 of SEQ ID NO:3 wherein nucleotide 120 is a G residue, thus indicating the presence of a HaeIII site. The X probe represented by SEQ ID NO:5 and the Y probe represented by SEQ ID NO:6 hybridize to opposite strands of the product.

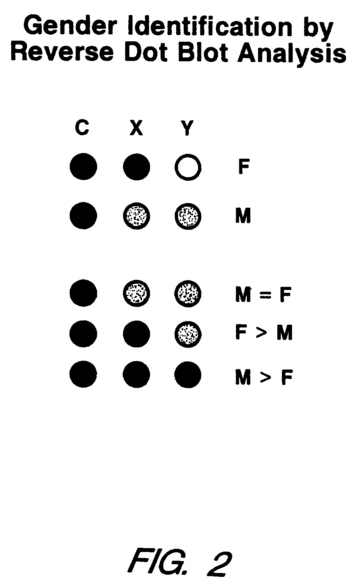

The probes are then exposed to the avidin horseradish peroxidase and the colorless dye. If any product has hybridized to any of the three probes, then those probes will be visualized by conversion of the colorless dye to a colored precipitate. As shown in Fig. 2, the degree of precipitation at each probe site permits the observer to distinguish samples in which:

    (1) only female DNA is present;

    (2) only male DNA is present;

    (3) female and male DNA are present in about an equal amount;

(4) a greater amount of female DNA than male DNA is present; and

(5) a greater amount of male DNA than female DNA is present.

The gender identification system of the present invention has numerous advantages over other systems. First, a product is made from both the X and Y chromosomes. Several other systems utilize Y specific sequences only, so that the absence of the Y product could be due to something other than the presence of female DNA; for example a PCR inhibitor may be present thus, resulting in a false female identification.

Second, in the present invention, the same primers are used to generate both X and Y products and these amplified products are the same size. Other systems involve multiple primer pairs, which may have different amplification efficiencies, or one primer pair that produces products of different length, which may be subject to preferential amplification of the smaller product. These types of systems may yield ambiguous results.

Third, the target sequence of the present invention (the 209 bp conserved region) is present in a single copy on each chromosome so relative amounts of X and Y sequence can be quantitated. Some other systems use primers within X- and Y-specific repeat sequences to boost the signal, but the number of the repeat sequences is variable and quantitation is not possible.

One minor disadvantage of some embodiments of the present invention is that a restriction digestion step may be required after amplification to determine the sex while some other systems can be typed directly on a gel following amplification. However, this disadvantage is overcome by utilizing the present invention in the reverse dot blot format. Then, the present invention could be added to an existing typing strip such as that present in the Roche Amplitype™ HLA DQα Forensic DNA Amplification and Typing Kit which would then allow direct typing.

The primer pair used in the present invention (RR10 and RR12) works under DQα conditions and can be co-amplified with DQα simply by biotinylating the primers and adding them to the Roche AmpliType™ DQα premix. Briefly, as explained in greater detail above, the C or control dot would have a probe to the region containing the HaeIII site common to the X and Y chromosomes or any other non-polymorphic region on SEQ ID NO:3. The X and Y dots would have probes complementary to the polymorphic HaeIII site region.

PCR products generated from a female DNA sample would hybridize to the C and X dots only and give dots of equal intensity. In contrast, PCR products generated from a male DNA sample would hybridize to the C, the X and the Y dots. The X and Y dots would be of equal intensity and half as dark as the C dots under ideal conditions.

Since these relative intensities can be achieved, it is possible to distinguish mixed samples, which give these types of dot patterns, from unmixed samples. Furthermore, it is possible to distinguish mixtures containing equal amounts of female and male DNA (because of the 3:1 X:Y ratio) from other mixtures.

An additional advantage of utilizing the present invention in the DQα Amplitype™ system is the ability to distinguish mixed casework samples with greater confidence. Specifically, there are several examples where the same DQα type appears in both the epithelial cell (female) fraction and the sperm fraction isolated from sexual assault evidence. However, a user of the present invention can legitimately make the claim that male DNA is present in both fractions if the Y chromosome dot appears on the same strip used to type the epithelial cell fraction. Also, in cases where the victim and assailant share a DQα type, the type in the sperm fraction can be assigned to the assailant if the Y chromosome dot is present.

The following examples are offered by way of illustration only and are by no means intended to limit the scope of the claimed invention.

Unless otherwise specified, percentages given herein for solids in solid mixtures, liquids in liquids, and solids in liquids are on a wt/wt, vol/vol and wt/vol basis, respectively. Furthermore, unless otherwise specified, the suppliers of reagents and instruments mentioned are not meant to be mandatory. The skilled person is in a position to select similar reagents or instruments from other suppliers.

Example 1

Gender Identification Assay Utilizing PCR With the RR10/RR12 Primer Pair

The following protocol describes the steps for PCR amplification of specific zfx and zfy regions of the X and Y chromosomes and the restriction enzyme digestion assay used to determine the gender of biological sample donors. All general and PCR-specific sample handling precautions should be taken.

I. PCR Amplification

Each 50 ml amplification reaction contained:

$$5\ \mu l \quad 10 \times \text{reaction mix} \qquad \text{) pre-mix and}$$
$$0.5\ \mu l \quad 10\ \mu M\ \text{RR10 (SEQ ID NO:1)} \quad \text{) add } 6\ \mu l \text{ to}$$
$$0.5\ \mu l \quad 10\ \mu M\ \text{RR12 (SEQ ID NO:2)} \quad \text{) each tube}$$

$$44\ \mu l \quad \text{DNA sample} + H_2O\ (\leq 15\ \mu l$$
$$\text{Chelex extracted DNA)}$$

The volume of 10 x reaction mix needed was determined (e.g. 20 reactions / 100 $\mu l$ 10 x reaction mix). A fresh mix for each assay was prepared using the following recipe for 100 $\mu l$ 10 x reaction mix (can increase or decrease total volume by maintaining proportions of components):

| $\mu l$ | |
|---|---|
| 25 | distilled $H_2O$ |
| 25 | 2 M KCl |
| 25 | 0.1 M $MgCl_2$ |
| 10 | 1M Tris-HCl, pH 8.3 |
| 10 | 18.8 mM dNTPs (each) |
| 5 | Taq DNA Polymerase (5 U/ml) |
| 100 | |

The amplification reactions were set up with all components except the DNA sample. Two drops of mineral oil were added and then the DNA sample was added through the oil. A negative control reaction (no added DNA) was also set up.

The samples were amplified as follows:

32 cycles:  94°C, 1 minute;
60°C, 1 minute;
72°C, 1 minute;
+10 minutes at 72°C

II. Restriction Enzyme Digestion

Each 12 $\mu l$ HaeIII digestion reaction contained:

$$10\ \mu l \quad \text{RR10/RR12 PCR product} + H_2O\ (5\text{-}10\ \mu l\ \text{PCR}$$
$$\text{product)}$$
$$1.2\ \mu l \quad 10 \times \text{HaeIII buffer (NEB)} \qquad \text{) pre-mix and}$$
$$0.3\ \mu l \quad H_2O \qquad\qquad\qquad\qquad\quad \text{) add } 2\ \mu l \text{ to}$$
$$0.5\ \mu l \quad \text{HaeIII (10 U/}\mu l;\ \text{NEB)} \qquad \text{) each reaction}$$

All of the components were set up in 0.5 ml tubes in a designated PCR product area. The digestion reactions were then incubated overnight at 37°C.

III. Visualization on Gel

The tubes were spun briefly to collect liquid and then 2 µl sample dye loading buffer was added. The entire reaction was loaded on a 9 % acrylamide gel (run bromophenol blue [BPB] dye 10 cm from origin). The gel was run in 1 x TBE. One uncut sample was included on this gel to indicate complete product digestion.

The RR10/RR12 amplified sample showed bands at 172 bp and 37 bp and a doublet at 88 to 84 bp following HaeIII digestion. Thus, it was concluded that the sample was from a male.

Example 2

Assay of Samples Containing Mixtures of Female and Male DNA

The procedures of Example 1 were used to analyze mixtures of female and male DNA. The following ratios of female to male DNA and male to female DNA were tested:

1:1
2,5:1
5:1
10:1
20:1
50:1
100:1.

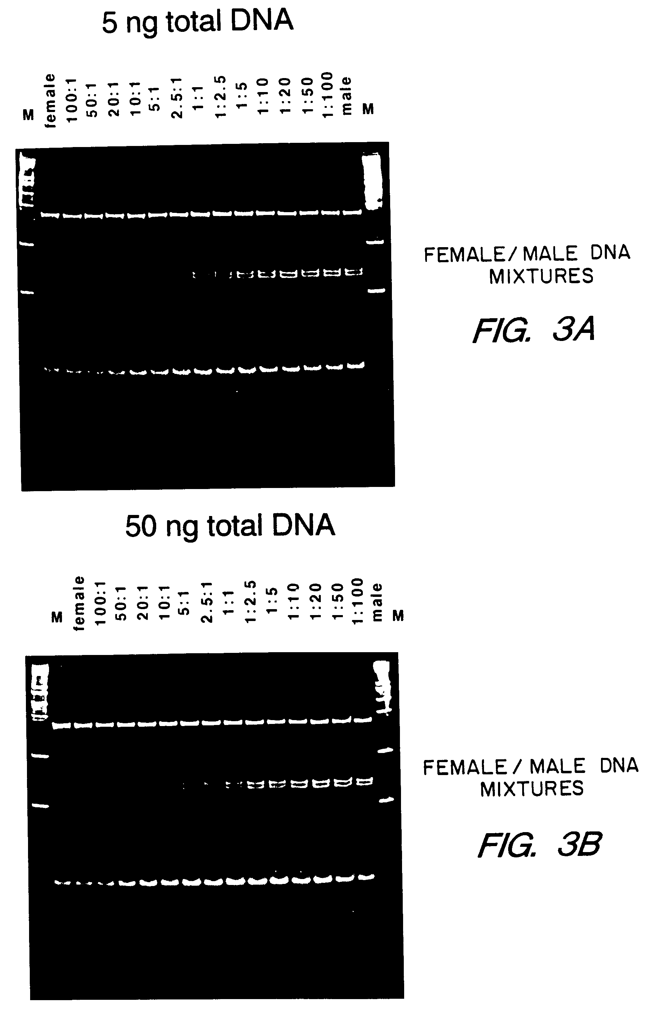

The total amount of DNA for each ratio was 5 ng and 50 ng in separate experiments. Following visualization on the 9 % acrylamide gel (see Fig. 3), it was found that the presence of male DNA could be detected by the presence of the 88/84 bp doublet up to the point where the male DNA component was present at 1/10 the amount of the female DNA.

Example 3

Assay of Male Sample Mixed With Female DNA

The RR10 (SEQ ID NO:1) and RR12 (SEQ ID NO:2) primers are fluorescently labelled using well-known conventional techniques. These fluorescently-tagged primers are then added to a mixed male DNA and female DNA sample in the PCR amplification protocol described in Example 1. Restriction enzyme digestion of the amplified products is also conducted in accordance with the procedures described in Example 1. The digested fluorescently-tagged products are visualized by a gene scanner, and the amounts of 88/84 bp doublet and 172 bp fragment are quantitated to reveal a female DNA component present at 1/4 the amount of male DNA.

Example 4

Reverse Dot Blot Gender Determination Assay

The desired 209 bp conserved region of a human DNA sample is amplified as explained in Example 1 except that the RR10 and RR12 primers have been biotinylated. The primers are biotinylated using the methods described by Innis, M.A. et al. in "PCR Protocols: A Guide to Methods and Applications", Academic Press, Inc. (1990). Specifically, 1 ml of 4 M lithium chloride is added to about 0.8 µmol of primer. The mixture is sonicated briefly and then transferred to a 1.5 ml Eppendorf tube and spun for about 2 minutes in a microfuge. The supernatant is filtered through a 0.45 µm syringe filter into a 12 ml silanized glass screw-cap tube. Then, 5 ml of a 1:1 mixture of cold absolute alcohol and acetone is mixed with the filtered supernatant and chilled for one hour in a freezer. The mixture is centrifuged at 4000 x g for 10 to 15 minutes, and the supernatant is discarded. Using sulfo-N- hydroxysuccinimide esters, a solution of 75 µmol of LC-NHS-biotin from Pierce Chemical Co. dissolved in 1 ml of 0.1 M sodium phosphate buffer (pH 7.6) is added to the pellet. The mixture is agitated to dissolve the pellet, and the solution is allowed to sit overnight. Excess biotin is then removed by pipetting the reaction mixture onto the top of a Sephadex™ G-25 column that is pre-equilibrated with 20 ml of 0.1 M triethylammonium acetate (TEAA) (pH 7.4). The eluate is then collected following application of 1.5 ml of 0.1 M TEAA to the column. The labelled primers are then purified by high pressure liquid chromotography (HPLC) using a PRP-1 column (7 by 305 mm, The Hamilton Co., Reno NV, U.S.A.) with a guard cartridge (Alltech Associates, Inc., Deerfield IL, U.S.A.) at a flow rate of 2 ml/minute with two solvents:

Solvent A - 0.1 M TEAA (pH7.4) containing 5 % acetonitrile

Solvent B - acetonitrile.

The gradient for the HPLC is:

30 minutes for equilibration;

5 to 10 % Solvent B over 30 minutes; and

10 to 50 % Solvent B over 10 minutes.

Ultra-violet radiation at 260 mm is used to detect the biotinylated primers which elute as large peaks at about 25 minutes after unmodified primers.

The X and Y chromosome products are visualized on nylon membrane strips containing immobilized probes, under the same hybridization conditions as DQα. These hybridization techniques and conditions are well-known to those skilled in the art, and taught in the product literature for Roche's Amplitype™ HLA DQα Forensic DNA Amplification and Typing Kit. Specifically, the amplified product is hybridized to the probe strips by incubation in a shaking water bath at 55°C for about 20 minutes at 50 to 90 rpm. A color development solution is then applied to the probe strips to cause the visualization of the product which has hybridized to the probe. A usefal color development solution comprises:

- 10 ml citrate buffer (0.1 M sodium citrate, pH 5.0)
- 10 ml 3 % hydrogen peroxide; and
- 0.5 ml chromogen solution

per probe strip.

The citrate buffer is prepared by dissolving 18.4 g trisodium citrate dihydrate in 800 ml deionized water. The pH of the solution is adjusted to 5.0 by addition of about 6 g citric acid monohydrate, and the final volume of buffer is adjusted to 1 liter by addition of deionized water.

A suitable chromogen solution is prepared by slowly adding 30 ml of room temperature 100 % ethanol to 60 mg of room temperature 3,3',5,5'-tetramethylbenizidine (TMB) powder. The mixture is then shaken for 30 minutes on an orbital shaker to dissolve the TMB. This solution is stored at 2 to 8°C, and is stable for four months after dissolution.

The color development of the Y probe dot on the probe strip indicates the presence of male DNA in the sample.

The foregoing written specification is considered to be sufficient to enable one skilled in the art to practice the invention. Various modifications of the invention in addition to those shown and described herein will become apparent to those skilled in the art from the foregoing description and fall within the scope of the appended claims.

SEQUENCE LISTING

(1) GENERAL INFORMATION:

    (i) APPLICANT:
        (A) NAME: F.HOFFMANN-LA ROCHE AG
        (B) STREET: Grenzacherstrasse 124
        (C) CITY: Basel
        (D) STATE: BS
        (E) COUNTRY: Switzerland
        (F) POSTAL CODE (ZIP): CH-4002
        (G) TELEPHONE: (0)61 - 688 24 03
        (H) TELEFAX: (0)61 - 688 13 95
        (I) TELEX: 962292/965542 hlr ch

    (ii) TITLE OF INVENTION: Gender determination method

   (iii) NUMBER OF SEQUENCES: 6

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.25 (EPO)

    (vi) PRIOR APPLICATION DATA:
        (A) APPLICATION NUMBER: US 07/883,660
        (B) FILING DATE: 15-MAY-1992

(2) INFORMATION FOR SEQ ID NO: 1:

    (i) SEQUENCE CHARACTERISTICS:
        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

   (iii) HYPOTHETICAL: NO

   (iii) ANTI-SENSE: NO

    (vi) ORIGINAL SOURCE:
        (A) ORGANISM: Homo sapiens

    (xi) SEQUENCE DESCRIPTION: SEQ ID NO: 1:

CTGGAGAGCC ACAAGCTGAC                     20

EP 0 569 833 A2

(2)  INFORMATION FOR SEQ ID NO: 2:

    (i)  SEQUENCE CHARACTERISTICS:
        (A)  LENGTH: 20 base pairs
        (B)  TYPE: nucleic acid
        (C)  STRANDEDNESS: single
        (D)  TOPOLOGY: linear

    (ii)  MOLECULE TYPE: DNA (genomic)

    (iii)  HYPOTHETICAL: NO

    (iii)  ANTI-SENSE: NO

    (vi)  ORIGINAL SOURCE:
        (A)  ORGANISM: Homo sapiens


    (xi)  SEQUENCE DESCRIPTION: SEQ ID NO: 2:

TTGCTGTGGA CTGCCAAGAG                          20

(2)  INFORMATION FOR SEQ ID NO: 3:

    (i)  SEQUENCE CHARACTERISTICS:
        (A)  LENGTH: 209 base pairs
        (B)  TYPE: nucleic acid
        (C)  STRANDEDNESS: single
        (D)  TOPOLOGY: linear

    (ii)  MOLECULE TYPE: DNA (genomic)

    (iii)  HYPOTHETICAL: NO

    (iii)  ANTI-SENSE: NO

    (vi)  ORIGINAL SOURCE:
        (A)  ORGANISM: Homo sapiens


    (xi)  SEQUENCE DESCRIPTION: SEQ ID NO: 3:

CTGGAGAGCC ACAAGCTGAC CAGCAAGGCA GAGAAGGCCA TTGAATGTGA TGAGTGTGGG     60

AAGCATTTTT CTCATGCAGG GGCTTTGTTT ACTCACAAAA TGGTGCATAA GGAAAAAGGG     120

GCCAACAAAA TGCACAAGTG TAAATTCTGT GAATATGAGA CAGCTGAACA GGGGTTATTG     180

AATCGCCACC TCTTGGCAGT CCACAGCAA                          209

(2)  INFORMATION FOR SEQ ID NO: 4:

    (i)  SEQUENCE CHARACTERISTICS:
        (A)  LENGTH: 19 base pairs
        (B)  TYPE: nucleic acid
        (C)  STRANDEDNESS: single
        (D)  TOPOLOGY: linear

12

```
        (ii)  MOLECULE TYPE: DNA (genomic)

        (iii) HYPOTHETICAL: NO

        (iii) ANTI-SENSE: NO

        (vi)  ORIGINAL SOURCE:
              (A) ORGANISM: Homo sapiens


        (xi)  SEQUENCE DESCRIPTION: SEQ ID NO: 4:

    AGAGAAGGCC ATTGAATGT                                    19

    (2) INFORMATION FOR SEQ ID NO: 5:

        (i)   SEQUENCE CHARACTERISTICS:
              (A) LENGTH: 20 base pairs
              (B) TYPE: nucleic acid
              (C) STRANDEDNESS: single
              (D) TOPOLOGY: linear

        (ii)  MOLECULE TYPE: DNA (genomic)

        (iii) HYPOTHETICAL: NO

        (iii) ANTI-SENSE: NO

        (vi)  ORIGINAL SOURCE:
              (A) ORGANISM: Homo sapiens


        (xi)  SEQUENCE DESCRIPTION: SEQ ID NO: 5:

    GAAAAAGGAG CCAACAAAAT                                   20

    (2) INFORMATION FOR SEQ ID NO: 6:

        (i)   SEQUENCE CHARACTERISTICS:
              (A) LENGTH: 20 base pairs
              (B) TYPE: nucleic acid
              (C) STRANDEDNESS: single
              (D) TOPOLOGY: linear

        (ii)  MOLECULE TYPE: DNA (genomic)

        (iii) HYPOTHETICAL: NO

        (iii) ANTI-SENSE: NO

        (vi)  ORIGINAL SOURCE:
              (A) ORGANISM: Homo sapiens

        (xi)  SEQUENCE DESCRIPTION: SEQ ID NO: 6:

    ATTTTGTTGG CCCCTTTTTC                                   20
```

**Claims**

1. A method for determining the gender of a human being based on a DNA sample originating from said human being, which method comprises the steps of:

(a) amplifying a product from the sample using a polymerase chain reaction procedure with the primers
CTGGAGAGCCACAAGCTGAC (SEQ ID NO:1); and
TTGCTGTGGACTGCCAAGAG (SEQ ID NO: 2);
(b) digesting the product with a HaeIII restriction enzyme; and
(c) visualizing the digested product.

2. The method of claim 1 wherein the amplified product is an approximately 209 base pair product.

3. The method of claim 2 wherein the amplified product has the nucleotide sequence

```
CTGGAGAGCC ACAAGCTGAC CAGCAAGGCA GAGAAGGCCA TTGAATGTGA
TGAGTGTGGG AAGCATTTTT CTCATGCAGG GGCTTTGTTT ACTCACAAAA
TGGTGCATAA GGAAAAAGGG GCCAACAAAA TGCACAAGTG TAAATTCTGT
GAATATGAGA CAGCTGAACA GGGGTTATTG AATCGCCACC TCTTGGCAGT
CCACAGCAA
```

[SEQ ID No: 3].

4. The method of claim 1 wherein the visualization is conducted on an acrylamide gel.

5. The method of claim 4 where in the gel shows a doublet band of approximately 88 and 84 base pairs, thus indicating the presence of male DNA.

6. The method of claim 1 wherein the visualization is conducted on an agarose gel.

7. The method of claim 6 wherein the gel shows a single band of approximately 88 to 84 base pairs, thus indicating the presence of male DNA.

8. A method for determining the gender of a human being based on a DNA sample originating from said human being, which method comprises the steps of:
(a) amplifying a 209 base pair product designated by SEQ ID NO:3 from the sample;
(b) digesting the product with an HaeIII restriction enzyme; and
(c) visualizing the digested product.

9. The method of claim 8 wherein the amplification step is conducted using ligase chain reaction procedures.

10. The method of claim 8 wherein the amplification step is conducted using polymerase ligase chain reaction procedures.

11. The method of claim 8 wherein the visualization is conducted on an acrylamide gel.

12. The method of claim 8 wherein the visualization is conducted on an agarose gel.

13. The method of claim 11 or claim 12 wherein the gel shows a single band of approximately 88 to 84 base pairs, thus indicating the presence of male DNA.

14. A method for determining the gender of a human DNA sample comprising the steps of:
(a) amplifying a product from the sample using a polymerase chain reaction procedure with primers designated by SEQ ID NO:1 and SEQ ID NO:2 which have been labelled;
(b) exposing the amplified product to three probes, wherein one probe is complementary to a region of the product common to female and male samples, another probe is complementary to a region of the product found only on X chromosomes, and another probe is complementary to a region of the product found only on Y chromosomes; and

(c) visualizing any product which has hybridized to the probes.

**15.** The method of claim 14 wherein the three probes are
AGAGAAGGCC ATTGAATGT (SEQ ID No: 4);
GAAAAAGGAG CCAACAAAAT (SEQ ID No: 5); and
ATTTTGTTGG CCCCTTTTTC (SEQ ID No: 6); respectively.

**16.** The method of claim 14 wherein the product is designated by SEQ ID NO:3.

**17.** The method of claim 14 wherein the primers are labelled with biotin and the visualization step comprises exposure of any hybridized product to an avidin horseradish peroxidase conjugate and a colorless dye which is converted to a colored precipitate by the horseradish peroxidase.

**18.** An oligonucleotide primer designated by SEQ ID NO:1.

**19.** An oligonucleotide primer designated by SEQ ID NO:2.

**20.** An oligonucleotide primer as claimed in claim 18 or claim 19 characterized in that the primer is labeled, e.g. with biotin.

**21.** An oligonucleotide as claimed in any one of claims 18 to 20 as a diagnostic tool.

**22.** Use of an oligonucleotide primer as claimed in any one of claims 18 to 20 for determining the gender of a human being based on a DNA sample originating from said human being.

**23.** A test-kit for determining the gender of a human being based on a DNA sample originating from said human being, which test-kit comprises in a container an oligonucleotide primer as claimed in any one of claims 18 to 20 and preferably the reagents needed for the amplification reaction.

HaeIII DIGESTION OF zfx/zfy PRODUCTS

**U M F M F F F M M**

123 bp

*FIG. 1*

## Gender Identification by
## Reverse Dot Blot Analysis

FIG. 2

# 5 ng total DNA

FEMALE/MALE DNA
MIXTURES

*FIG. 3A*

# 50 ng total DNA

FEMALE / MALE DNA
MIXTURES

*FIG. 3B*